# EUROPEAN PATENT APPLICATION

(11) **EP 4 681 689 A1**
(43) Date of publication of application: **21.01.2026**
(21) Application number: 24189015.1
(22) Date of filing: 16.07.2024
(51) Int. Cl.: A61F 13/38, B08B 1/14, B08B 9/02, B08B 9/04

(54) **SWAB**

(71) Applicant: Illinois Tool Works Inc., Glenview IL 60025 (US)
(72) Inventor: SALVADOR, Jaymar, Glenview, 60025 (US); NGUYEN, Tan, Glenview, 60025 (US)
(74) Representative: HGF

(57) **Abstract**

According to the present invention, there is provide a swab (100). The swab (100) includes a head (102) and a handle (104). The head (102) has a curved outer surface (106). The handle (104) includes a head portion (114) and a holding portion (118). The holding portion (118) of the handle (104) has a planar outer surface (120). The head portion (114) of the handle (104) is at least partially received within the head (102) such that the holding portion (118) of the handle (104) extends outwardly from one end of the head (102). The swab (100) may be a cleaning swab and/or an applicator swab.

## Description

### BACKGROUND

The present invention relates to a swab. More particularly, but not exclusively, the present invention relates to a swab for cleaning and/or for applying substances to an inner surface of a tube or tubular component, for example the an inner surface of a tubular product.

### SUMMARY OF THE INVENTION

According to an aspect of the invention there is provided a swab including a head and a handle (which may also be referred to as a swab head and a swab handle). A portion of the handle, for example the swab handle, may have a planar outer surface.

The head, e.g. the swab head, may, for example, have a curved outer surface.

The handle, e.g. the swab handle, may include a head portion and a holding portion. The holding portion may, for example, include the planar outer surface.

The head portion of the handle may be at least partially received within the head such that the holding portion of the handle extends outwardly from one end of the head.

According to an aspect of the invention there is provided a swab including a head having a curved outer surface, and a handle. The handle includes a head portion and a holding portion. The head portion of the handle is at least partially received within the head such that the holding portion of the handle extends outwardly from one end of the head. The holding portion of the handle has a planar outer surface.

Swabs in accordance with the invention can be manipulated with ease such that they can be inserted into, and pulled through, the internal bore of tubing or tubular components, for example the internal bore of tubular products. Swabs according to the invention can, advantageously, be used to clean an inner surface of a tube or tubular component and/or to apply a substance to an inner surface of a tube or tubular component.

The head may have a first, closed, end and a second, open, end. The holding portion of the handle may therefore extend from the second, open end of the head. The head portion of the handle is at least partially received within the head. An end of the handle that forms part of the head portion may be positioned adjacent to the closed end of the head.

The head portion of the handle may be generally cylindrical to facilitate manufacture of the handle. The head portion of the handle may, for example, be coaxial with the head.

The holding portion of the handle may be generally cuboidal such that the swab can be manipulated with ease.

The planar outer surface of the holding portion may have a textured portion. The textured portion may include a pattern of straight, angled and/or crossed indentations in the handle portion. The textured portion may be, for example, knurled.

The holding portion of the handle may have an upper surface and an opposing lower surface. The textured portion may be provided on the upper surface of the holding portion of the handle. Additionally, or alternatively, the textured portion may be provided on the lower surface of the holding portion of the handle.

The holding portion of the handle may have first and second side surfaces. The textured portion may be provided on the first side surface and/or the second side surface of the holding portion.

In examples of the invention, the textured portion may be provided on any combination of the upper surface, the lower surface, the first side surface and the second side surface of the holding portion. In other words, the textured portion may be provided on one, two, three or four surfaces of a cuboidal holding portion.

The textured portion, for example the knurled upper surface and/or the knurled lower surface and/or the knurled first side surface and/or the knurled second side surface of the holding portion of the handle, facilitates manipulation of the handle and therefore the swab of which the handle forms a part.

The handle may include a tapered portion. The tapered portion may be, for example, intermediate the head portion and the holding portion of the handle. The tapered portion of the handle may be, for example, generally frustoconical.

The tapered portion of the handle provides strength to the handle without unnecessarily increasing the size or weight of the handle of the swab.

The head portion of the handle may have a head portion thickness. The head portion thickness may correspond, in examples of the invention, to a diameter of the generally cylindrical head portion of the handle.

The holding portion may have a holding portion thickness. The holding portion thickness may correspond, in examples of the invention, to a depth of the generally cuboidal holding portion of the handle.

The head portion thickness may be greater than the holding portion thickness.

The holding portion thickness may be up to 60% of the head portion thickness, for example up to 55% of the head portion thickness.

The holding portion thickness may be at least 5% of the head portion thickness, for example at least 10% of the head portion thickness.

The holding portion thickness may be between 5% and 60% of the head portion thickness, for example between 5% and 55% of the head portion thickness.

The holding portion thickness may be between 10% and 60% of the head portion thickness, for example between 10% and 55% of the head portion thickness.

The holding portion thickness may be approximately 45% of the head portion thickness.

The configuration of the head portion of the handle advantageously provides a secure connection between the head and the handle of the swab without unnecessarily increasing the size or weight of the handle of the swab.

The head of the swab may have a head thickness. The head thickness may correspond, in examples of the invention, to the diameter of a generally cylindrical head of a swab. The head thickness may be greater than the head portion thickness of the handle such that the head portion of the handle can be accommodated or housed within the head of the swab.

The head portion thickness may be up to 60% of the head thickness, for example up to 50% of the head thickness.

The head portion thickness may be at least 5% of the head thickness, for example at least 15% of the head thickness.

The head portion thickness may be between 5% and 60% of the head thickness, for example between 5% and 50% of the head thickness.

The head portion thickness may be between 15% and 60% of the head thickness, for example between 15% and 50% of the head thickness.

The head portion thickness may be approximately 47% of the head thickness.

The swab may have a swab length. The handle may have a handle length. The head portion of the handle may have a head portion length. The holding portion of the handle may have a holding portion length.

The holding portion length may be up to 50% of the swab length, for example up to 40% of the swab length, for example up to 30% of the swab length, for example up to 25% of the swab length.

The holding portion length may be at least 5% of the swab length, for example at least 15% of the swab length, for example at least 20% of the swab length.

The holding portion length may be between 5% and 50% of the swab length, for example between 5% and 40% of the swab length, for example between 10% and 30% of the swab length, for example between5% and 25% of the swab length.

The holding portion length may be between 15% and 50% of the swab length, for example between 15% and 40% of the swab length, for example between 15% and 30% of the swab length, for example between 15% and 25% of the swab length.

The holding portion length may be between 20% and 50% of the swab length, for example between 20% and 40% of the swab length, for example between 20% and 30% of the swab length, for example between 20% and 25% of the swab length.

The head of the swab may have a head length. The head length may be, for example, defined between the first end and the second end of the head of the swab.

A ratio of the head length to the holding portion length may be less than 6.0, for example less than 4.5, for example less than 3.0.

A ratio of the head length to the holding portion length, for example the ratio of the head length to the holding portion length may be at least 0.5, for example at least 1.0, for example at least 2.0.

The ratio of the head length to the holding portion length may be between 1.0 and 6.0, for example between 1.0 and 4.5, for example between 1.0 and 3.0.

The ratio of the head length to the holding portion length may be between 2.0 and 6.0, for example between 2.0 and 4.5, for example between 2.0 and 3.0.

The ratio of the head length to the holding portion length may be approximately 2.7.

In embodiments of the invention, the head of the swab may include a neck portion. The neck portion may be at or adjacent to the open end of the head. The holding portion of the handle may extend outwardly from the neck portion of the head.

In embodiments of the invention, the tapered portion of the handle may extend outwardly from the neck portion of the head.

In embodiments of the invention, a portion of the head portion of the handle may extend outwardly from the neck portion of the head of the swab.

The swab may be an applicator swab. Additionally or alternatively, the swab may be a cleaning swab.

Other technical features may be readily apparent to one skilled in the art from the following figures, descriptions, and claims.

### BRIEF DESCRIPTION OF THE SEVERAL VIEWS OF THE DRAWINGS

An embodiment of the invention will now be described, by way of example only, hereinafter with reference to the accompanying drawings.

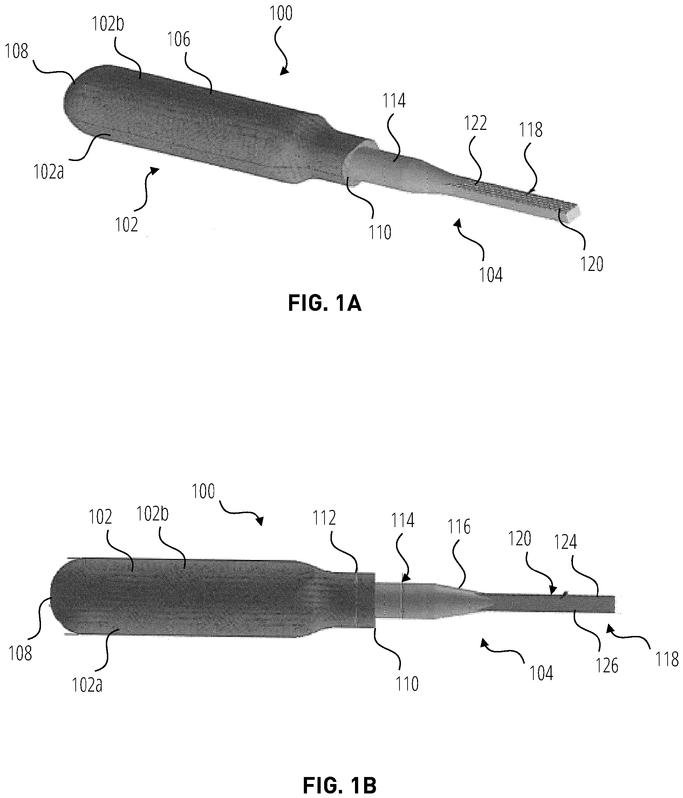

FIG. 1A illustrates a perspective view of a swab according to an embodiment of the invention;
FIG. 1B illustrates a side view of the swab of FIG. 1A; and

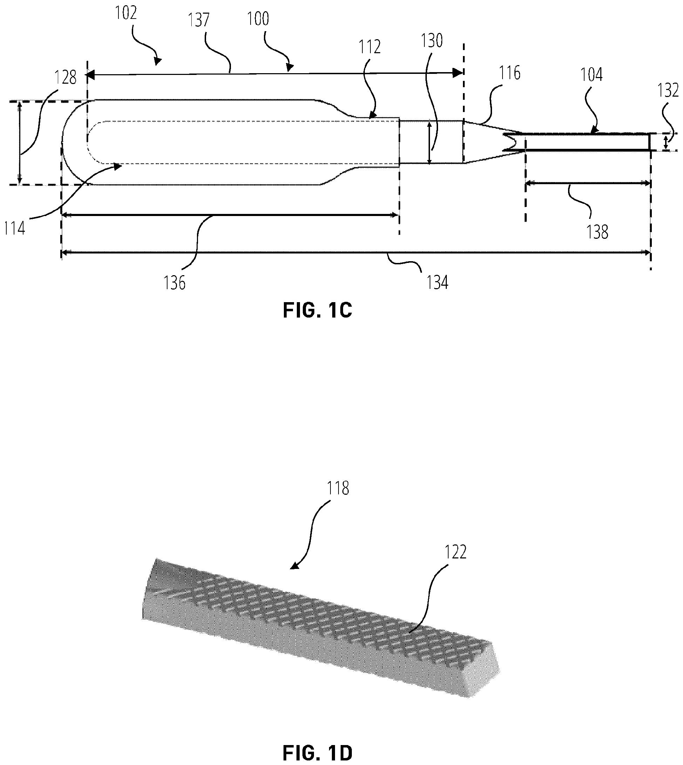

FIG. 1C illustrates a cross-section view of the swab of FIG. 1A.
FIG. 1D illustrates a a view of the holding portion of the handle of FIG. 1A.

### DETAILED DESCRIPTION

Certain terminology is used in the following description for convenience only and is not limiting. The words 'right', 'left', 'lower', 'upper', 'front', 'rear', 'upward', 'down' and 'downward' designate directions in the drawings to which reference is made and are with respect to the described component when assembled and mounted. The words 'inner', `inwardly', `outer' and 'outwardly' refer to directions toward and away from, respectively, a designated centreline or a geometric centre of an element being described (e.g. central axis), the particular meaning being readily apparent from the context of the description.

Further, as used herein, the terms 'connected', 'attached', 'coupled' and 'mounted' are intended to include direct connections between two members without any other members interposed therebetween, as well as, indirect connections between members in which one or more other members are interposed therebetween. The terminology includes the words specifically mentioned above, derivatives thereof, and words of similar import.

Further, unless otherwise specified, the use of ordinal adjectives, such as, "first", "second" and "third" etc. merely indicate that different instances of like objects are being referred to and are not intended to imply that the objects so described must be in a given sequence, either temporally, spatially, in ranking or in any other manner.

Like reference numerals are used to depict like features throughout.

Referring now to FIG. 1A, FIG. 1B, FIG. 1C and FIG. 1D, there is a swab 100 in accordance with an embodiment of the invention. It will be appreciated that the swab 100 may be an applicator swab and/or a cleaning swab.

The swab 100 includes a head 102 (a swab head) and a handle 104 (a swab handle).

The swab head 102 has a first swab head portion 102a and a second swab head portion 102b. Together, the first swab head portion 102a and the second swab head portion 102b form a generally cylindrical swab head 102. In other words, each of the first swab head portion 102a and the second swab head portion 102b has a curved outer surface such that the first swab head portion 102a and the second swab head portion 102b together form a curved outer surface 106 of the swab head 102. The head 102 has a first, closed end 108 and a second, open end 110. A neck portion 112 of the head 102 is provided adjacent to the open end 110 of the swab head. The neck portion 112 is also generally cylindrical, but has a smaller outer diameter than the head 102 of the swab 100.

The handle 104 is an elongate structure and has a head portion 114, a tapered portion 116 and a holding portion 118. The head portion 114 is generally cylindrical. The tapered portion 116 is generally frustoconical. The holding portion 118 is generally cuboidal. The holding portion 118 thus has a planar outer surface 120.

As shown in FIG. 1A and FIG. 1D, the planar outer surface 120 of the holding portion 118 has a textured portion 122. The textured portion may be formed by any suitable process including, for example, knurling.

With particular reference to FIG. 1B it can be seen that the holding portion 118 has an upper surface 124 and a lower surface 126. Although the textured portion 122 is only visible on the upper surface 124 (in FIG. 1A and FIG. 1D), it will be appreciated that, in embodiments of the invention, the textured portion 122 may be provided on any surface of the holding portion 118, for example on the upper surface 124 and/or the lower surface 126 and/or a first side surface and/or a second side surface of the holding portion 118.

The generally frustoconical tapered portion 116 of the handle 104 is intermediate the head portion 114 and the holding portion 118 of the handle 104.

Referring now to FIG. 1C, it can be seen that the that the head 102 of the swab 100 has a head diameter or thickness 128, that the head portion 114 of the handle 104 has a head portion diameter or thickness 130, and that the holding portion 118 has a holding portion thickness 132.

The swab head diameter or thickness 128 may be at least 2.0 mm, for example at least 5.5 mm.

The swab head diameter or thickness 128 may be less than or equal to 20.0 mm, for example less than or equal to 7.0 mm.

The swab head diameter or thickness 128 may be between 2.0 mm and 20.0 mm, for example between 2.0 mm and 7.0 mm.

The swab head diameter or thickness 128 may be between 5.5 mm and 20.0 mm, for example between 5.5 mm and 7.0 mm.

The head portion diameter or thickness 130 may be at least 1.0 mm, for example at least 2.75 mm.

The head portion diameter or thickness 130 may be less than or equal to 10.0 mm, for example less than or equal to 3.25 mm.

The head portion diameter or thickness 130 may be between 1.0 mm and 10.0 mm, for example between 1.0 mm and 3.25 mm.

The head portion diameter or thickness 130 may be between 2.75 mm and 10.0 mm, for example between 2.75 mm and 3.25 mm.

The holding portion thickness may be at least 0.5 mm, for example at least 1.25 mm.

The holding portion thickness may be less than or equal to 3.0 mm, for example less than or equal to 1.5 mm.

The holding portion thickness may be between 0.5 mm and 3.0 mm, for example between 0.5 mm and 1.5 mm.

The holding portion thickness may be between 1.25 mm and 3.0 mm, for example between 1.25 mm and 1.5 mm.

In examples of the invention, the swab head diameter or thickness 128 may be between 2 mm and 20 mm, the head portion diameter or thickness 130 may be between 1 mm and 10 mm, and the holding portion thickness 132 may be between 0.5 mm and 3 mm.

In one example of the invention, the swab head diameter or thickness 128 may be between 5.5 mm and 7.0 mm, the head portion diameter or thickness 130 may be between 2.75 mm and 3.25 mm, and the holding portion thickness 132 may be between 1.25 mm and 1.5 mm.

It can also be seen that the swab 100 has a swab length 134, that the head 102 has a head length 136, and that the holding portion 118 of the handle 104 has a holding portion length 138.

The swab length 134 may be any length.

The swab length 134 may be at least 20 mm, for example at least 45 mm.

The swab length 134 may be less than or equal to 5000 mm, for example less than or equal to 3000 mm, for example less than or equal to 1000 mm, for example less than or equal to 500 mm, for example less than or equal to 250 mm, for example less than or equal to 200 mm, for example less than or equal to 100 mm, for example less than or equal to 78 mm.

The swab length 134 may be between 20 mm and 5000 mm, for example between 20 mm and 3000 mm, for example between 20 mm and 1000 mm, for example between 20 mm and 500 mm, for example between 20 mm and 250 mm, for example between 20 mm and 200 mm, for example between 20 mm and 100 mm, for example between 20 mm and 78 mm.

The swab length 134 may be between 45 mm and 5000 mm, for example between 45 mm and 3000 mm, for example between 45 mm and 1000 mm, for example between 45 mm and 500 mm, for example between 45 mm and 250 mm, for example between 45 mm and 200 mm, for example between 45 mm and 100 mm, for example between 45 mm and 78 mm.

The swab head length 136 may be at least 2 mm, for example at least 26 mm. The swab head length 136 may be less than or equal to 4500 mm, for example less than or equal to 3000 mm, for example less than or equal to 2000 mm, for example less than or equal to 1000 mm, for example less than or equal to 500 mm, for example less than or equal to 250 mm, for example less than or equal to 200 mm, for example less than or equal to 100 mm, for example less than or equal to 40 mm, for example less than or equal to 28 mm.

The swab head length 136 may be between 2 mm and 4500 mm, for example between 2 mm and 3000 mm, for example between 2 mm and 2000 mm, for example between 2 mm and 1000 mm, for example between 2 mm and 500 mm, for example between 2 mm and 250 mm, for example between 2 mm and 200 mm, for example between 2 mm and 100 mm, for example between 2 mm and 40 mm, for example between 2 mm and 28 mm.

The swab head length 136 may be between 26 mm and 4500 mm, for example between 26 mm and 3000 mm, for example between 26 mm and 2000 mm, for example between 26 mm and 1000 mm, for example between 26 mm and 500 mm, for example between 26 mm and 250 mm, for example between 26 mm and 200 mm, for example between 26 mm and 100 mm, for example between 26 mm and 40 mm, for example between 26 mm and 28 mm.

The head portion length 137 may be at least 27 mm, for example at least 50 mm.

The head portion length 137 may be less than or equal to 4500 mm, for example less than or equal to 3000 mm, for example less than or equal to 2000 mm, for example less than or equal to 1000 mm, for example less than or equal to 500 mm, for example less than or equal to 250 mm, for example less than or equal to 200 mm, for example less than or equal to 100 mm, for example less than or equal to 80 mm, for example less than or equal to 55 mm.

The head portion length 137 may be between 27 mm and 4500 mm, for between 27 mm and 3000 mm, for example between 27 mm and 2000 mm, for example between 27 mm and 1000 mm, for example between 27 mm and 500 mm, for example between 27 mm and 250 mm, for example between 27 mm and 200 mm, for example between 27 mm and 100 mm, for example between 27 mm and 80 mm, for example between 27 mm and 55 mm.

The head portion length 137 may be between 50 mm and 4500 mm, for between 50 mm and 3000 mm, for example between 50 mm and 2000 mm, for example between 50 mm and 1000 mm, for example between 50 mm and 500 mm, for example between 50 mm and 250 mm, for example between 50 mm and 200 mm, for example between 50 mm and 100 mm, for example between 50 mm and 80 mm, for example between 50 mm and 55 mm.

The holding portion length 138 may be at least 5 mm, for example at least 9.5 mm.

The holding portion length 138 may be less than or equal to 4500 mm, for example less than or equal to 3000 mm, for example less than or equal to 2000 mm, for example less than or equal to 1000 mm, for example less than or equal to 500 mm, for example less than or equal to 250 mm, for example less than or equal to 200 mm, for example less than or equal to 100 mm, for example less than or equal to 50 mm, for example less than or equal to 25 mm, for example less than or equal to 20.5 mm.

The holding portion length 138 may be between 5 mm and 4500 mm, for between 5 mm and 3000 mm, for example between 5 mm and 2000 mm, for example between 5 mm and 1000 mm, for example between 5 mm and 500 mm, for example between 5 mm and 250 mm, for example between 5 mm and 200 mm, for example between 5 mm and100 mm, for example between 5 mm and 50 mm, for example between 5 mm and 25 mm, for example between 5 mm and 20.5 mm.

The holding portion length 138 may be between 9.5 mm and 4500 mm, for between 9.5 mm and 3000 mm, for example between 9.5 mm and 2000 mm, for example between 9.5 mm and 1000 mm, for example between 9.5 mm and 500 mm, for example between 9.5 mm and 250 mm, for example between 9.5 mm and 200 mm, for example between 9.5 mm and100 mm, for example between 9.5 mm and 50 mm, for example between 9.5 mm and 25 mm, for example between 9.5 mm and 20.5 mm.

In examples of the invention, the swab length 134 may be between 20 mm and 200 mm. The swab head length 136 may be between 2 mm and 40 mm, the head portion length 137 may be between 27 mm and 80 mm and the holding portion length 138 may between 5 mm and 200 mm.

In one example of the invention, the swab length may be between 45 mm and 78 mm, the swab head length 136 may be between 26 mm and 28 mm, the head portion length 137 may be between 50 mm and 55 mm. and the holding portion length 138 may be between 9.5 mm and 20.5 mm.

In embodiments of the invention, the handle 104 may be manufactured from a polymeric material, for example a thermoplastic material, e.g. polypropylene. The head 102 may be manufactured from a foam material, for example polyurethane foam, e.g. an open-cell polyurethane. The open-cell polyurethane foam may, in an example of the invention be a 39.4 pores per centimetre (100 pores per inch) foam.

The swab head 102 is formed from a first foam head portion 102a and a second foam head portion 102b, each of which may be moulded.

The handle 104 is made by a moulding method. In one example of the invention, the textured portion 122 is provided on the holding portion 118 of the handle 104 using a knurling method in which a patterned roller is pressed onto and moved over the surface to be textured and an imprint of the pattern is formed on the handle portion. In alternative examples of the invention, the textured pattern may be formed on the outer surface 120 of the holding portion of the handle during manufacture, for example during moulding, of the handle 104.

The handle 104 is then sandwiched between the first and second foam head portions 102a, 102bsuch that the head portion 114 of the handle 104, or at least a portion of it, is housed between the first foam head portion 102a and the second foam head portion 102b of the head 102 and the tapered portion 116 and holding portion 118 of the handle 104 extend outwardly from the head 102.

The first and second foam head portions 102a, 102b are bonded together using any suitable means, for example heating or an ultrasonic bonding method, to form the swab head 102.

Similarly, the head 102 and the handle 104 of the swab 100 are bonded together using any suitable means, for example heating or an ultrasonic bonding method, to form the swab 100.

In an alternative embodiment of the invention, the swab head 102 may be formed as a generally cylindrical body having an aperture at the open end 110 into which the head portion 114 of the handle 104 is inserted. The swab head 102 and the handle 104 may then be bonded together using any suitable means, for example heating or an ultrasonic bonding method, to form the swab 100.

To use the swab 100, a user holds the holding portion 118 of the handle 104 and pulls the swab 100 through a tube or tubular component or product such that the swab head 102 is pulled along the inner bore of the tube or tubular component or product.

Prior to use, the swab head 102 may be soaked in a cleaning solution such that, as the swab head 102 is pulled along the inner bore of the tube or tubular component or product, the swab head 102 and the cleaning solution clean the inner bore. Such a swab 100 may be referred to as a cleaning swab.

The swab head 102 may be soaked in other materials or substances, for example coatings or lubricants, such that as the swab head 102 is pulled along the inner bore of the tube or tubular component or product, the material or substance, for example the coating or lubricant, is applied to the inner bore of the tube or tubular component or product. Such a swab 100 may be referred to as an applicator swab.

It will be appreciated by persons skilled in the art that the above detailed examples have been described by way of example only and not in any limitative sense, and that various alterations and modifications are possible without departing from the scope of the invention as defined by the appended claims. Various modifications to the detailed examples described above are possible.

Through the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract or drawings), or to any novel one, or any novel combination, of the steps of any method or process so disclosed.

### LIST OF FEATURES

- 100: Swab
- 102: Swab head
- 102a: First swab head portion
- 102b: Second swab head portion
- 104: Handle
- 106: Curved outer surface
- 108: Closed end
- 110: Open end
- 112: Neck portion
- 114: Head portion
- 116: Tapered portion
- 118: Holding portion
- 120: Planar outer surface
- 122: Textured portion
- 124: Upper surface
- 126: Lower surface
- 128: Swab head thickness
- 130: Head portion thickness
- 132: Holding portion thickness
- 134: Swab length
- 136: Swab head length
- 137: Head portion length
- 138: Holding portion length

## Claims

1. A swab comprising:
a head having a curved outer surface; and
a handle comprising a head portion and a holding portion;
wherein the head portion of the handle is at least partially received within the head such that the holding portion of the handle extends outwardly from one end of the head; and
wherein the holding portion has a planar outer surface.

2. The swab of claim 1, wherein the head portion of the handle is generally cylindrical.

3. The swab of claim 1 or 2, wherein the holding portion of the handle is generally cuboidal.

4. The swab of any one of claims 1 to 3, wherein the planar outer surface of the holding portion has a textured portion.

5. The swab of claim 4, wherein the textured portion is knurled.

6. The swab of claim 4 or 5, wherein the holding portion of the handle has an upper surface and an opposing lower surface, and wherein the textured portion is provided on one or both of the upper surface and the lower surface of the holding portion of the handle.

7. The swab of any one of claims 1 to 6, wherein the handle comprises a tapered portion intermediate the head portion and the holding portion.

8. The swab of claim 7, wherein the tapered portion of the handle is generally frustoconical.

9. The swab of any one of claims 1 to 8, wherein the head portion has a head portion thickness and the holding portion has a holding portion thickness, wherein the head portion thickness is greater than the holding portion thickness.

10. The swab of claim 9, wherein the head has a head thickness, and the head thickness is greater than the head portion thickness of the handle.

11. The swab of any one of claims 1 to 10, wherein the swab has a swab length and the holding portion has a holding portion length, and wherein the holding portion length is up to 50% of the swab length.

12. The swab of claim 11, wherein the head has a head length, and wherein a ratio of the head length to the holding portion length is at least 0.5.

13. The swab of claim 12, wherein the ratio of the head length to the holding portion length is less than 6.0.

14. The swab of any one of claims 1 to 13, wherein the head comprises a neck portion from which the holding portion of the handle extends outwardly.

15. The swab of claim 14, when dependent on claim 7, wherein the tapered portion of the handle extends outwardly from the neck portion of the head.
